# FASCICULE DE BREVET EUROPEEN

(11) **EP 0 819 134 B1**
(45) Date de publication et mention de la délivrance du brevet: **18.07.2001**
(21) Numéro de dépôt: 96911015.4
(22) Date de dépôt: 02.04.1996
(51) Int. Cl.: C07H 21/00, C12N 15/11, C07H 19/04

(54) **OLIGONUCLEOTIDES A LIAISON COVALENTE TRANSVERSALE, PROCEDE DE PREPARATION ET SYNTHON UTILE DANS LE PROCEDE**
OLIGONUKELOTIDE MIT TRANSVERSALKOVALENTER BINDUNG, VERFAHREN ZU IHRER HERSTELLUNG UND DARIN BENUTZTES SYNTHON
COVALENTLY CROSS-LINKED OLIGONUCLEOTIDES, METHOD FOR PREPARING SAME, AND SYNTHON USED THEREIN

(30) Priorité: 03.04.1995 FR 9503889
(43) Date de publication de la demande: 21.01.1998
(73) Titulaire: GENSET SA, 75008 Paris (FR)
(72) Inventeur: BLUMENFELD, Marta, F-75013 Paris (FR); MERENKOVA, Irena, F-75011 Paris (FR)
(74) Mandataire: Warcoin, Jacques
(86) Numéro de dépôt international: FR9600493
(87) Numéro de publication internationale: WO9631523

(56) Documents cités:
- WO-A-88/00201
- WO-A-92/19732
- WO-A-93/18052
- NUCLEOSIDES AND NUCLEOTIDES, vol. 11, no. 7, 1992, pages 1333-1351, XP002005505 F.BENSELER ET AL.: "Synthesis of Suitably-Protected Phosphoramidites of 2'-Fluoro-2'-Deoxyguanosine and 2'-Amino-2'-Deoxyguanosine for Incorporation into Oligoribonucleotides"
- BIOORG. KHIM., vol. 19, no. 4, 1993, pages 455-466, XP002005506 L.G.KUZNETSOVA ET AL.: "Oligodeoxyribonucleotides Containing 2'-Amino-2'-deoxypyrimidine Nucleosides"

## Description

La présente invention concerne des oligonucléotides duplexes à liaison(s) transversale(s) covalente(s), c'est-à-dires des duplexes d'ADN, d'ARN ou mixtes ADN/ARN dont les deux brins complémentaires sont liés transversalement de manière covalente.

Plus précisément la présente invention concerne un oligonucléotide simple brin à liaison(s) covalente(s) intra oligonucléotidique(s), c'est-à-dire un oligonucléotide simple brin qui comporte un liaison covalente entre les deux sites de l'oligonucléotide ou plusieurs liaisons covalentes entre un nombre multiple de deux de sites de l'oligonucléotide. En particulier, l'oligonucléotide peut être du type "en épingle à cheveux", c'est-à-dire comportant deux fragments d'un même brin appariés, séparés par une boucle non auto-appariée, la liaison covalente étant localisée dans lesdits fragments appariés.

La présente invention concerne également un oligonucléotide double brin à liaison(s) covalente(s) interoligonucléotidique(s), c'est-à-dire un oligonucléotide double brin dans lequel les deux brins appariés sont reliés l'un à l'autre par des liaisons covalentes entre un ou plusieurs sites de chacun desdits deux brins.

Des oligonucléotides de ce type ont été décrits dans la demande de brevet PCT WO93/18052. Ils sont utiles dans une application thérapeutique, notamment dans la stratégie dite "sens" où ils sont utilisés pour leur propriété à s'hybrider à des facteurs impliqués dans la transcription de gènes impliqués dans des pathologies. Des agents sens de ce type ont été décrits également dans la demande de brevet PCT WO92/19732.

Les oligonucléotides à liaison(s) transversale(s) sont également utiles comme réactifs de recherche à des fins d'élucidation du mécanisme d'interaction ADN-protéine. En particulier lorsque cette interaction est liée à une perturbation de la structure de la double-hélice (ARN polymérase, méthylase, uracylglycosilase et d'autres enzymes de réparation).

L'utilisation pratique de duplexes à liaison transversale permet l'étude, dans des systèmes in vitro du niveau d'expression génique dans différents tissu (lorsque des facteurs spécifiques prennent part à ce mécanisme) et constitue un moyen efficace pour l'inhibition de la transcription et sa modulation.

Ces duplexes ont comme propriété particulièrement avantageuse d'être plus résistants aux endonucléases et exonucléases que des oligonucléotides duplexes sans liaison covalente.

Un but de la présente invention est de fournir des oligonucléotides à liaison transversale covalente améliorée, c'est-à-dire que la liaison covalente doit être suffisamment flexible pour se conformer avec la structure hélicoïdale du duplexe ou d'autres structures secondaires et tertiaires de l'ADN.

Un autre but de la présente invention est de fournir un procédé simplifié de préparation de ces oligonucléotides à liaison covalente transversale.

Un but supplémentaire de la présente invention est d'obtenir une liaison covalente qui peut être coupée sans déshybrider les oligonucléotides ou parties d'oligonucléotides lié(e)s de façon covalente.

Dans le document WO93/18052, il a été décrit des duplexes à liaison covalente transversale dans lesquels la liaison covalente résulte de la réaction d'un groupe aldéhyde, plus précisément d'un groupe di-aldéhyde et d'un groupe amine. Cette réaction fournit une base de schiff, suivie d'une réduction par un borohydrure dont résulte un lien covalent hydrazide. Il est également décrit une réaction entre un aldéhyde et des groupes hydroxyles qui résulte en un lien acétal.

La présente invention consiste à utiliser une liaison covalente de type amide 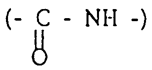 entre un groupe amine primaire et un groupe carboxyle. Ce type de lien permet d'atteindre les buts fixés par la présente invention.

Bien que ce type. de liaison amide soit avantageux car la chimie en est beaucoup plus simple, une simple réaction de condensation en présence d'un réactif de condensation du type carbodiimide est nécessaire, il n'a pas été décrit dans le document WO93/18052 pour la raison suivante. Ce type de liaison amide, lorsqu'il implique deux acides nucléiques, plus précisément deux oligonucléotides, doit être réalisé dans un milieu acide à pH d'environ 6,5. Or le pKa d'un groupe amine primaire aliphatique est de l'ordre de 11. Dans les conditions de pH réactionnel de l'ordre de 6,5, le groupe amine primaire aliphatique se trouve donc majoritairement sous forme protonée et la forme nucléophile de l'amine est quasiment inexistante, de sorte que la réaction ne peut pas avoir lieu, ou en tous cas à très faible rendement.

Toutefois, selon la présente invention, on a tiré parti du fait qu'un groupe àmine situé en position 2' d'un 2'-désoxynucléotide possède un pKa nettement inférieur, à savoir de l'ordre de 7,4, ce qui autorise le couplage de cet amine avec un groupe carboxyle dans des conditions de pH compatibles avec le couplage de deux oligonucléotides. Du fait du niveau de pH réactionnel, il se maintient davantage sous forme libre non-protonée restant ainsi nucléophile.

Une caractéristique essentielle de la présente invention est donc d'utiliser un nucléotide modifié portant un groupe NH₂ en position 2' d'un 2'-désoxynucléotide couplé à un autre nucléotide modifié ou analogue de nucléotide modifié, portant un groupe aliphatique ayant à son extrémité un groupe carboxyle.

Plus précisément la présente invention a pour objet un oligonucléotide double brin ou simple brin comportant une ou plusieurs liaison(s) covalente(s) transversale(s) respectivement inter ou intra oligonucléotidique(s) caractérisé en ce que la ou chaque liaison covalente est constituée par un lien amide entre un groupe amine primaire d'un brin et un groupe carboxyle de l'autre brin ou du même brin respectivement, ledit groupe amine primaire étant substitué directement en position 2' du cycle osidique d'un nucléotide d'un brin et, ledit groupe carboxyle étant porté par un groupe aliphatique espaceur substitué sur un nucléotide ou un analogue de nucléotide de l'autre brin ou du même brin respectivement.

On entend par "analogue de nucléotide", un insert non-nucléotidique qui comporte, comme les nucléotides naturels un chaînon linéaire de 3 atomes de carbone entre les deux liaisons internucléotidiques, de sorte que ledit chainon a pour squelette : 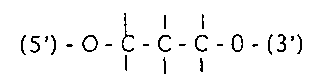 Cet insert non-nucléotidique permet de respecter la distance inter-nucléotidique naturelle. Ledit "analogue de nucléotide" se présente sous la forme d'un reste monovalent ou d'un reste divalent s'il remplace un nucléotide terminal ou respectivement un nucléotide non terminal dans l'oligonucléotide.

Le groupe aliphatique compris dans la liaison covalente a un rôle de groupe espaceur car sa taille doit être telle que la liaison covalente entre les deux oligonucléotides concernés comprenne une chaine linéaire dont le squelette comporte de 5 à 25 atomes alignés, de sorte que la distance entre les deux brins d'une part, et la conformation spaciale de la double hélice d'autre part, soient respectées et permettent une bonne hybridation entre les deux brins.

Dans un mode de réalisation, ledit groupe carboxyle est porté par un analogue de nucléotide consistant en un insert non-nucléotidique du type propane-diol-1,3 substitué en position 2 par un groupe aliphatique(R) portant à son extrémité ledit groupe carboxyle.

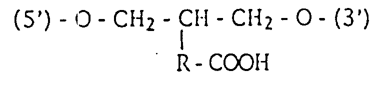

Dans un autre mode de réalisation, ledit groupe carboxyle est porté par un groupe aliphatique substitué en position 2' d'un 2' désoxynucléotide.

Toutefois, il est préférable d'utiliser un analogue de nucléotide car les réactions sont plus faciles à réaliser dans la mesure où il n'y a pas lieu de protéger le nucléotide dans les différents autres groupes fonctionnels du cycle osidique et/ou de la base purique ou pyrimidique.

Le(s) nucléotide(s) et analogue de nucléotide le cas échéant impliqués dans une liaison covalente inter ou intra moléculaire peuvent être en position d'appariement, c'est-à-dire face à face sur chaque brin des fragments appariés.

Toutefois selon la taille dudit groupe aliphatique il peut être préférable dans certains cas que le(s) nucléotide(s) et analogue de nucléotide impliqués dans une liaison covalente ne soient pas en position d'appariement et soient décalés de 1 ou plusieurs nucléotides par rapport à la position d'appariement. Ainsi, de façon appropriée, le(s) nucléotide(s) et analogue de nucléotide impliqués dans une liaison covalente sont décalés de 1 à 5 nucléotides par rapport à la position d'appariement.

Avantageusement, ledit groupe aliphatique comporte une chaîne linéaire de 3 à 23 atomes alignés entre ledit lien amide et le site du nucléotide ou analogue de nucléotide sur lequel il est substitué, ce qui correspond à une distance entre le(s) nucléotide(s) et/ou analogue de nucléotide impliqués dans la liaison, de 5 à 25 atomes comme mentionné ci-dessus.

De préférence encore, ledit groupe aliphatique comporte de 7 à 16 atomes.

Dans un mode de réalisation, ledit groupe aliphatique comporte 9 atomes et le nucléotide et analogue de nucléotide impliqués dans la liaison covalente sont décalés de 2 nucléotides par rapport à la position d'appariement.

Ledit groupe aliphatique espaceur substitué par un groupe carboxyle, peut lui-même résulter de l'acylation par un anhydride d'acide 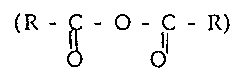 d'un groupe amine primaire substitué directement en position 2' d'un nucléotide ou en position 2 d'un analogue de nucléotide.

Dans une variante avantageuse, ledit groupe aliphatique espaceur substitué par un groupe carboxyle résulte de l'acylation d'un groupe amine substitué directement en position 2' sur un nucléotide ou sur un analogue de nucléotide en position 2, par un acide aminé dont l'extrémité NH₂ est elle-même acylée par un anhydride d'acide.

En particulier on cite l'exemple où l'acide aminé est la β-alanine.

De façon appropriée, l'anhydride d'acide est un anhydride cyclique tel que l'anhydride succinimique ou l'anhydride tartarique.

Dans le cas où la liaison covalente comporte une β-alanine couplée à un anhydride succinimique ou tartarique, un reste en position 2' d'un 2' désoxynucléotide d'un brin est relié au reste en position 2 d'un analogue du type 1,3 propane diol d'un autre brin ou du même brin par un reste divalent respectivement de formule 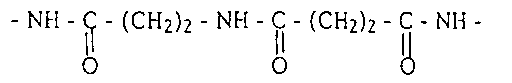 ou

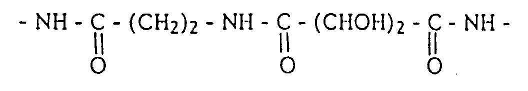

De façon avantageuse, l'anhydride d'acide comporte un groupe cis-glycol. En effet, ce groupe glycol peut être clivé dans des réactions d'oxydation douce sans déshybrider le duplexe.

Un autre avantage des groupes amine en position 2' d'un 2'-désoxynucléotide ou en position 2 d'un analogue de nucléotide, notamment du type propane-diol-1,3, est la possibilité d'obtenir des synthons qui peuvent être mis en oeuvre dans des procédés de synthèse d'oligonucléotides conventionnels avec le groupe NH₂ en position 2' protégé notamment par un groupe trifluoroacétyle 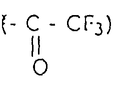 notamment dans les réactions de synthèse automatique au phosphoramidite.

Ainsi une ou plusieurs modification(s) peut(vent) être introducite(s) dans un oligonucléotide directement en cours de synthèse dans n'importe quel site prédéterminé de la chaine oligonucléotidique à l'aide d'un synthon monomérique approprié.

La présente invention a donc également pour objet un synthon nucléotidique utile dans la préparation d'un oligonucléotide selon la présente invention, caractérisé en ce qu'il comporte les extrémités 5' et 3' convenablement protégées pour être utilisé dans un procédé de synthèse automatique ou manuelle d'acides nucléiques et un groupe amine substitué en position 2' protégé par un groupe trifluoroacétyle.

Ce groupe trifluoroacétylc peut être ensuite sélectivement éliminé en vue du couplage de l'oligonucléotide le comprenant, avec un anhydride d'acide.

La présente invention a en outre pour objet un synthon non nucléotidique utile dans la préparation d'un oligonucléotide selon la présente invention, caractérisé en ce qu'il est constitué par un composé propane-diol dont les fonctions hydroxyle en positions 1 et 3 sont protégées par les groupes protecteurs conventionnels des fonctions 5' et 3' de nucléotides dans les réactions de synthèse d'oligonucléotide et la position 2 est substituée directement par un groupe NH₂ ou un reste acyl d'acide aminé dont le groupe NH₂ libre est protégé par un groupe trifluoroacétyle.

Lesdits groupes hydroxyle sont protégés par des groupes protecteurs utilisés dans les procédés de synthèse d'oligonucléotides au phosphoramidite.

Dans un mode de réalisation utile pour une synthèse au phosphoramidite, le synthon nucléotidique répond à la formule :

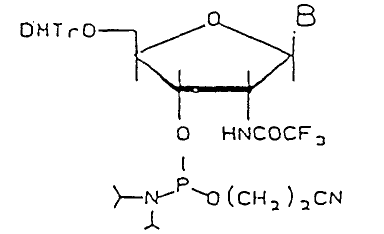

Dans un mode de réalisation, le synthon non nucléotidique répond à la formule : 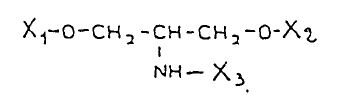 dans laquelle X₁ et X₂ sont des groupes protecteurs conventionnels des groupes OH 5' et 3' d'un nucléotide, utilisés dans les procédés de synthèse d'oligonucléotide. X₃ est un groupe protecteur de groupe amine ou un reste acyl d'acide aminé dont le groupe NH₂ est protégé par un groupe protecteur de groupe amine.

En particulier, on utilise un synthon de formule :

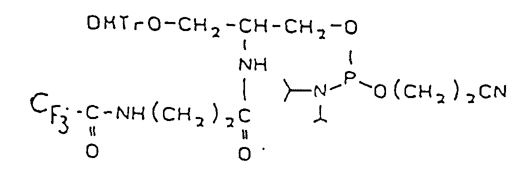

Enfin la présente invention a pour objet un procédé de préparation d'un oligonucléotide selon l'invention, caractérisé en ce qu'on réalise une liaison covalente de type amide entre un groupe amine primaire d'un brin et un groupe carboxyle de l'autre brin ou d'une autre partie du même brin, par réaction de condensation à pH 6,5 en présence d'un agent de condensation.

De façon appropriée, l'agent de condensation est du carbodiimide soluble dans l'eau.

De façon avantageuse, on prépare le brin comportant un groupe carboxyle à partir d'un brin comportant un groupe amine obtenu à l'aide de synthons nucléotidiques conventionnels et d'un synthon selon la présente invention que l'on couple à un anhydride d'acide comportant undit groupe aliphatique.

Le principe de la synthèse chimique des acides nucléiques sur support solide est, aujourd'hui, largement décrit dans la littérature spécialisée, et un certain nombre d'appareils sont disponibles sur le marché qui éxécutent automatiquement tout ou partie des étapes de la synthèse. Parmi les voies chimiques décrites, la méthode dite des phosphoramidites (EP 0 035 719 B1, EP 61746) présente à ce jour une efficacité suffisante pour la production à l'échelle industrielle des acides nucléiques.

D'autres caractéristiques et avantages de la présente invention apparaitront à la lumière de la description détaillée suivante.

Des oligonucléotides (A) et (B) ont été préparés en suivant la procédure standard de la synthèse oligonucléotidique au phosphoramidite.

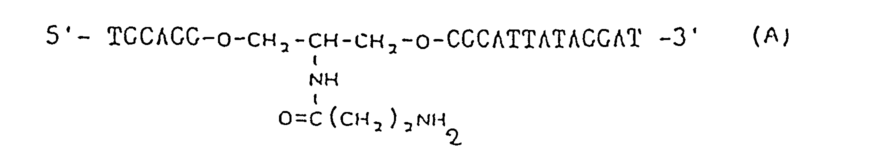

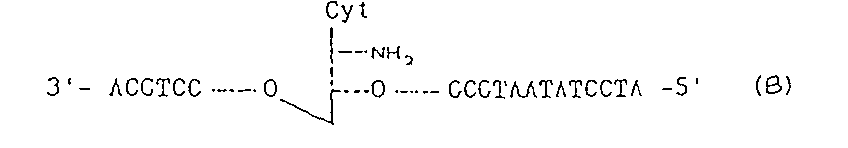

Puis, on a réalisé le couplage de (A) avec l'anhydride succinimique pour obtenir (C) :

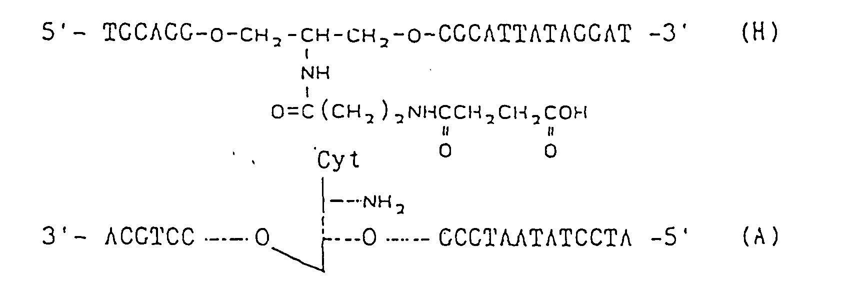

Puis on a réalisé le couplage des deux brins (B) et (C) pour obtenir le duplexe suivant :

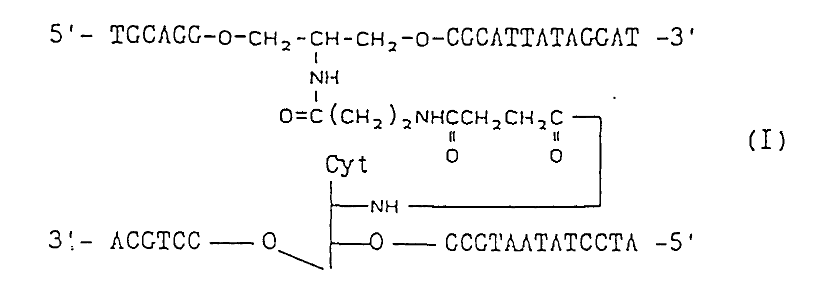

A cette fin les nucléosides 3'-phosphoramidites 2'-amino-2'-désoxycytidine (2) et 2'-amino-2'-désoxyuridine (1), d'une part et la phosphoramidite de l'insert non-nucléolidique, d'autre part, ont été synthétisés.

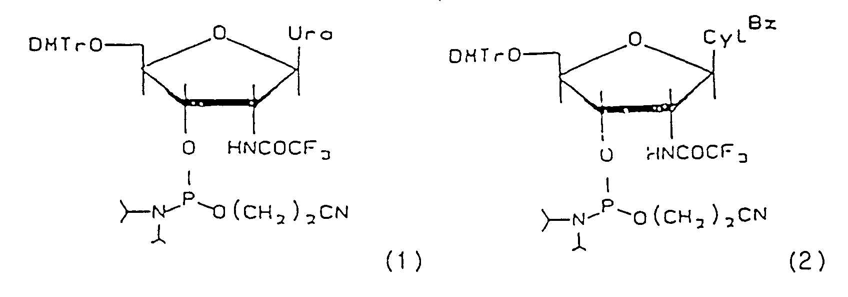

Un groupe amine aliphatique initial, localisé sur un insert non-nucléosidique (3) a été introduit dans le second brin du duplexe cible. Le phosphoramidite correspondant (4) a été synthétisé à partir d'un ester actif de β-alanine bloquée par N-Fmoc (5) et du dérivé di-méthoxytrityle (Dmtr) du 2-amino-1, 3-propanédiol (6) (schéma 1) en procédant comme suit :
- condensation des composés (5) et (6) dans du dioxane pour produire le composé cible (3) ;
- phosphitylation du composé (3) avec le N,N-diisopropylamido-β-cyanoéthylchlorophosphite pour former le synthon phosphoramidite (4).

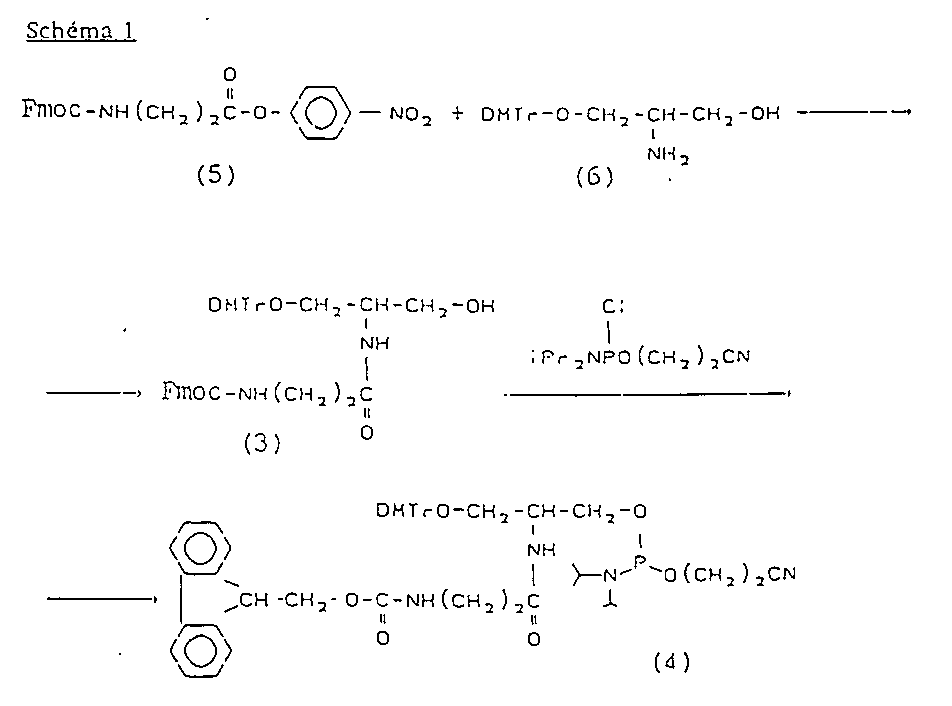

La synthèse de l'ester p-nitrophényle de β-alanine bloquée par N-FMOC (5) est donnée dans le schéma 2 et a été réalisée selon les étapes suivantes :
- blocage du groupe amine de la β-alanine (7) en utilisant la protection Fmoc par la réaction avec la Fmoc-succinimide (8),
- obtention de l'ester activé de β-alanine bloquée par N-Fmoc (5) par l'action du p-nitrophénol sur (9) à l'aide de dicyclohexylcarbodiimide présent dans du dioxane.

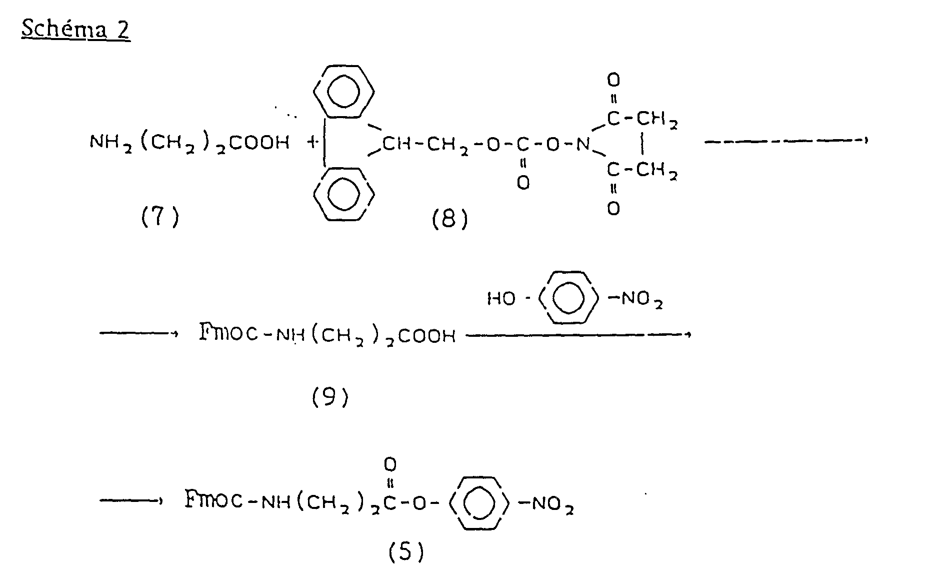

La préparation du 1-O-diméthoxytrityle-2-amino-propanédiol (6) est représentée sur le schéma 3 et a impliqué les réactions suivantes :
- blocage de la fonction amine primaire du 2-amino-1, 3-propanédiol (10) à l'aide du groupe trifluoroacétyle,
- protection de chacun des deux groupes hydroxyles du composé (11) par l'action du chlorure de diméthoxytrityle dans de la pyridine (1 mol pour 0,5 mol de composé (11)).
- élimination de la protection par le trifluoroacétyle par l'action d'une solution concentrée d'hydroxyde d'ammonium.

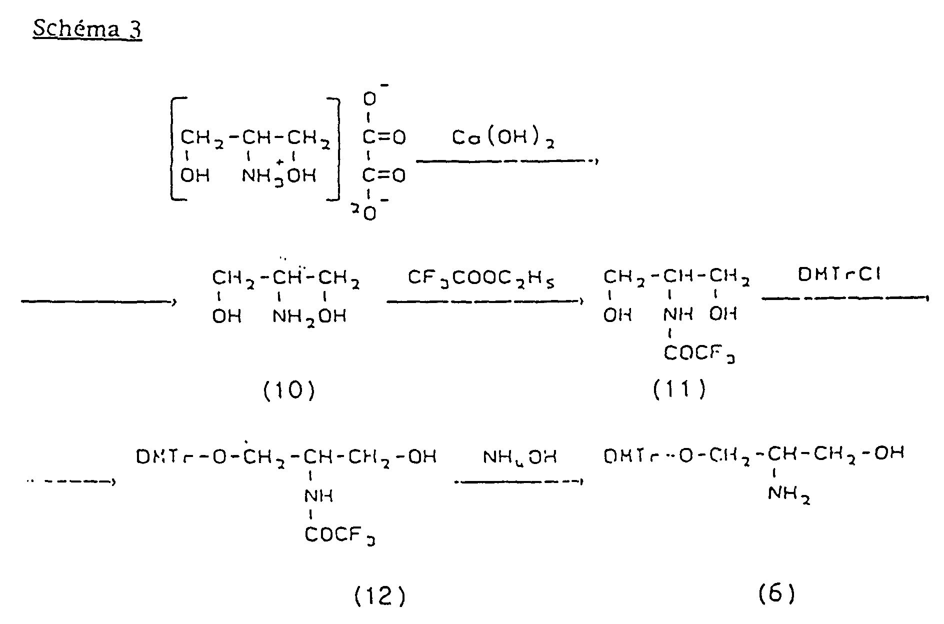

Le composé cible (4), contenant le groupe amine aliphatique, a été introduit de manière directionnelle au niveau d'un site, défini précisément, d'une chaîne oligonucléotidique dans une procédure standard de synthèse d'oligonucléolide dite au phosphoramidite. Cet analogue non-nucléosidique maintient la distance de 3 atomes de carbone (caractéristique des nucléosides naturels) entre les hydroxyles formant la liaison internucléotidique. En utilisant le composé (4) de nombreux oligonucléotides modifiés ont été obtenus. On préfère toutefois utiliser un groupe protecteur trifluoracétyle car le TFA est plus facile à éliminer sélectivement que le groupe Fmoc. Il s'est révélé que le groupe amine primaire aliphatique ainsi inséré dans les oligonucléotides modifiés est hautement réactif dans des réactions ayant des réactifs électrophiles. Ainsi, on a réalisé une réaction avec l'anhydre acétique suivie par l'analyse HPLC à paires d'ions des produits de la réaction ainsi que la réaction avec le fluorescéïnisothiocyanate (FITC) suivie par l'analyse spectrophotométrique des produits. Le spectre du produit résultant de la réaction avec le FITC a révélé deux pics d'absorption à 260 et à 495 nm (rapport 6:1) correspondant à l'oligonucléotide et la fluorescéine, respectivement.

De plus, le groupe carboxyle a été introduit dans les oligonucléotides modifiés contenant la fonction amine, par la réaction de condensation sur (4) avec l'anhydride succinimique. Le rendement de réaction en des produits résultant de la carboxylation des oligonucléotides avec l'anhydride succinimique a été supérieure à 95 %.

Il doit être noté que le temps de rétention des oligonucléotides modifiés analysés par HPLC à paires d'ions a varié avec la nature des groupes fonctionnels introduits. Ainsi le temps de rétention des oligonucléotides, possédant le groupe amine, était inférieur à celui des oligonucléotides naturels possédant une structure primaire analogue. Ceci était dû au fait que de tels oligonucléotides avaient une charge positive supplémentaire. Le temps de rétention des oligonucléotides possédant le groupe carboxyle était au contraire supérieur du fait d'une charge négative supplémentaire.

La fonction amine localisée sur l'insert non-nucléosidique de l'oligonucléotide a aussi été acylée par l'anhydride tartarique. Le duplex à liaison transversale synthétisé en utilisant un tel précurseur contenant un carboxyle, possède une liaison de type cis-glycol. Dans ce duplexe à liaison transversale il est possible de séparer les chaînes du duplexe (clivé la liaison entre les chaînes) par une réaction d'oxydation.

Les exemples suivants illustrent la présente invention.

### EXEMPLE 1: Synthèse Standard d'Oligonucléotides

Les synthèses oligonucléotidiques ont été réalisées sur le synthétiseur 380B d'Applied Biosystems suivant les protocoles et les cycles standards au phosphoramidite en utilisant des réactifs fournis par le fabricant. Lorsque des phosphoramidites modifiés ont été utilisées, un temps plus long de couplage (5 à 7 min.) a été utilisé. Les oligonucléotides ont été normalement synthétisés à une échelle de 0,4 µmol dans le mode "Trytil-On". Les conditions standards de déprotection (ammonium 30 %, 55°C, 16h) ont été utilisées.

L'HPLC a été réalisée sur un Altex équipé d'un détecteur Kratos UV. Les oligonucléotides ont été analysés et purifiés par chromatographie liquide haute pression en phase inverse sur une colonne Diasorb 130 C16_{T} (7 micron) (4 mm ID x 250 mm). Le solvant A était du AcONH₄ (acétate d'ammonium) 0,1 M, pH 7,0, le solvant B était du AcONH₄ 0,1 M, pH 7,0 acétonitrile 40 % (V/V). Le gradient linéaire de 0% à 100 % de B a été appliqué en 60 min. L'éluat a été contrôlé à 260 nm ou à 290 nm.

Pour le résultat de l'analyse réactionnelle, une HPLC à paires d'ions a été utilisée avec une étape de 2 résidus par min. Un système de gradient HPLC Waters, une colonne 4 x 250 mm remplie avec Diasorb 130 C16_{T} (7 micron), un éluant : phosphate-K 50 mM (pH 7,0) avec du tétrabutylammonium 2 mM et un gradient d'acétonitrile de 5 à 40 %, un débit de 1 ml/min., une température de 45°C ont été utilisés.

Toutes les séquences oligonucléotidiques sont représentées sur la liste dans un ordre standard 5' vers 3' de gauche à droite. Le préfixe "d" (désoxy) est omis dans l' identification des 2'-désoxyribonucléosides et des oligodésoxyribonucléotides.

### EXEMPLE 2 : Préparation de nucléotides activés et protégés et d'inserts non-nucléosidiques ayant un groupe espaceur et un groupe fonctionnel actif.

Une chromatographie couche mince (TLC) a été réalisée sur des plaques Kieselgel 60 F₂₅₄ (Merck), la chromatographie sur colonne a été réalisée sur Kieselgel 60 (Merck) en utilisant un des systèmes de solvant suivants a) CHCl₃/EtOH 95:5 (v/v) ; b) CHCl₃/EtOH 9:1 (v/v) ; c) CH₂Cl₂/CH₃OH/E-t₃N 94:5:1 (v/v/v), les spectres UV ont été obtenus sur un spectrophotomètre 150-20 (Hitachi, Japon).

Le 5'-0-(4, 4'-Dimétoxytrityle)-2'-trifluoroacétamido-2'-désoxyuridine-3'-0-(β-cyanoéthyle-N,N-diisopropylamido)phosphite (1) et le 5'-0-(4,4'-Dimétoxytrityle)-N4-benzoyle-2'-trifluoroacétamido-2'désoxycytidine-3'-0-(βcyanoéthyle-N,N-diisopropylamido)-phosphite (2) ont été préparés selon Kuznetsova L.G., Romanova E.A., Volkov E.M., Tashlitsky V.N., Oretskaya T.S., Krynetskaya N.F., Shabarova Z.A. Bioorganicheskaya khimia (Russ.) 1993, 19, 455-466.

Préparation de 2-amino-1, 3-propanédiol (10). Le 2-amino-1, 3-propanédiol oxalate (3,0 g, 11 mmol) a été dissous dans 100 ml d'eau. L'hydroxyde de calcium (0,82 g, 11 mmol) a été ajouté sous agitation. Après 30 min. le précipité a été filtré et le surnageant a été concentré sous vide. On obtient 1,9 g (95 %).

La trifluoroacétylation de (10) a été réalisée selon le procédé, décrit dans Kuznetsova L.G., Romanova E.A., Volkov E.M., Tashlitsky V.N., Oretskaya T.S., Krynetskaya N.F., Shabarova Z.A. Bioorganicheskaya khimia (Russ.) 1993, 19, 455-466.

La tritylation du 2-trifluoroacétamido-1, 3-propanédiol (11) a été réalisée en utilisant la procédure similaire destinée aux nucléosides : Jones R.A., Oligonucleotide synthesis : a Practical Approach. Ed. by M.J. Gait. Oxford, Washington DC : IRL Press. 1984, 23-24.

Préparation du 1-Dimétoxytrityle-2-aminopropanol-3 (6). Le composé (12) (2,94 g, 6 mmol) a été dissous dans 30 ml de EtOH et du NH₄OH (18 ml) a été ajouté goutte à goutte sous agitation. Le mélange réactionnel a été chauffé jusqu'à 45°C pendant 2 heures et les solvants ont été éliminés sous vide. Le résidu a été purifié sur une colonne de gel de silice éluée avec le système de solvant b) pour donner 1,73 g (73 %) (6) R_{f} 0,25 (système a).

Préparation de la N-fluorénylméthoxycarbonyle-β-alanine (9). Le 9-Fluorénylméthoxysuccinimide (8) (0,86 g, 2,56 mmol) a été ajouté goutte à goutte sous agitation à une solution de β-alanine (7) (0,46 g, 5,16 mmol) dans 10 ml d'eau à l'aide de 700 ml d'Et₃N. Après 30 min., le mélange a été concentré sous vide et le résidu a été ajouté à 8 ml de HCl 1,5 M. Le matériel insoluble a été éliminé par filtration, lavé deux fois à l'eau et séché à 45°C. Production de (9) 0,76 g (95 %), R_{f} 0,5 (système a).

Préparation de ether de N-fluorénylméthoxycarbonyle-β-alaninep-nitrophényle (5). Du p-Nitrophénol (0,40 g, 3,2 mmol) a été ajouté à (9) (0,90 g, 2,9 mmol) dissous dans 5 ml de dioxane déshydraté. Le mélange a été agité pendant 24 h à température ambiante. Le matériel insoluble a été éliminé par filtration. Le filtrat a été concentré sous vide jusqu'à ce qu'il soit sec. Production de (5) 1,2 g (95 %). R_{f} 0,8 (système a).

Le Composé (3) a été préparé à partir de (5) (1,2 g, 2,8 mmol) par la réaction avec (6) (1,14 g, 0,3 mmol) dans du dioxane déshydraté (10 ml). Le mélange a été agité pendant 24 h à température ambiante. Le solvant a été éliminé sous pression réduite, le résidu a été purifié sur une colonne de gel de silice avec le système de solvant c). On obtient 1,2 g de (3) (63 %). R_{f} 0,4 (système b).

¹H-RMN (CDCl₃) 6,1 (d, 1H, CH-NH-CO, J 7,9 Hz); 5,53 (t, 1H, CH₂-NH-CO, J 6,1 Hz); 4,35 (d, 2H, O-CH₂-CH, J 7,0 Hz) ; 4,18 (t, 1H, CH₂-CH, J 7,0 Hz) ; 4,1 (m, 1H, CH₂-CH-CH₂), 3,75 (s, 6H, CH₃O) ; 3,7 (2d, 2H, OCH₂-CH, J₁ 11,3 Hz, J₂ 4,8 Hz) ; 3,48 (m, 2H, CH₂-CH₂-NH) ; 3,3 (2d, 2H, CH-CH₂OH, J 11,3 Hz, J 4,0 Hz), 2,41 (m, 2H, CO-CH₂-CH₂).

La phosphitylation de (3) a été réalisée selon Jones R.A. Oligonucleotide synthesis : a Practical Approach. Ed. by M.J. Gait. Oxford, Washington DC : IRL Press. 1984.

### EXEMPLE 3 : Préparation d'Oligonucléotides ayant des nucléosides 2'-amino-2'-désoxypyrimidine

1. Oligonucléotide contenant un seul résidu de 2'-amino-2'-désoxycytidine
   L'oligonucléotide de séquence oligomère (A)
   ATC CTA TAA TGC GCⁿC CTG CA
   dans lequel Cⁿ représente un nucléotide 2'-amino-2'-désoxycytidine, a été préparé en utilisant la procédure de l'Exemple 1. La 2'-amino-2'-désoxycytidine située au milieu de la séquence a été ajoutée pendant la synthèse en utilisant la 2'-amino-2'-désoxycytidine phosphoramidite (2).
2. Oligonucléotides contenant un seul résidu 2'-amino-2'-désoxyuridine
   Lés oligonucléotides de séquence
   Oligomère (B) G CGA CTC GGA AAG TCC CCT CUⁿA CCG
   Oligomère (C) G CCA UⁿTC GGA AAG TCC CCT CTA CCG
   Oligomère ( D) G CCA CTC GGA AAG TGA GCT CUⁿA CCG
   Oligomère (E) G CCA UⁿTC GGA AAG TGA GCT CTA CCG
   dans lesquels Uⁿ représente un nucléotide 2'-amino-2'-désoxyuridine, ont été préparés en utilisant la procédure de l'Exemple 1. La 2'-amino-2'désoxyuridine située au milieu de la séquence a été ajoutée pendant la synthèse en utilisant la 2'-amino-2'-désoxyuridine phosphoramidite (1).
3. Oligonucléotides contenant des résidus multiples de 2'-amino-2'-désoxyuridine
   Les oligonucléotides de séquence
   Oligomère (F) G CCA UⁿTC GGA AAG TCC CCT CUⁿA CCG
   Oligomère (G) G CCA UⁿTC GGA AAG TGA CGT CUⁿA CCG
      dans lesquels Uⁿ représente une 2'-amino-2'-désoxyuridine, ont été préparés en utilisant la procédure de l'Exemple 3.2.

### EXEMPLE 4 : Préparation d'Oligonucléotides ayant une insertion non-nucléosidique

1. Oligonucléotides contenant une seule insertion non-nucléosidique
   Les oligonucléotides de séquence
   Oligomère (H) TCG AGG NGC GCA TTA TAG GAT
   Oligomère (I) CGG TNG AGG GGA CTT TCC GAG TGG C
   Oligomère (J) CGG TAG AGG GGA CTT TCC GAN TGG C
   Oligomère (K) CGG TNG AGC TCA CTT TCC GAG TGG C
   Oligomère (L) CGG TAG AGC TCA CTT TCC GAN TGG C
   Oligomère (M) CGG TAG NGG GGA CTT TCC GAG TCC C
      dans lesquels N représente un insert non-nucléosidique, ont été préparés en utilisant la procédure de l'Exemple 1. L'insert non-nucléosidique a été introduit dans la séquence pendant la synthèse en utilisant une phosphoramidite non-nucléosidique (4). Les oligonucléotides ont été préparés en utilisant une procédure standard au phosphoramidite. Ils ont été déprotégés par la procédure normale de déprotection, purifiés par HPLC et détritylés.
2. Oligonucléotides contenant de multiples insertions non-nucléosidiques
   Les oligonucléotides de séquence
   Oligomère (N) CGG TNG AGG GGA CTT TCC GAN TGG C
   Oligomère (O) CGG TNG AGC TCA CTT TCC GAN TGG C
      dans lesquels N représente un insert non-nucléosidique, ont été préparés en utilisant la procédure de l'Exemple 4.1.
3. La carboxylation du groupe amine localisé sur l'insert non-nucléosidique des oligomères (H) à (O) a été réalisée avec de l'anhydride succinimique dans un tampon de bicarbonate de sodium 0,25 M (pH 8). 1,0 unité d'A₂₆₀ d'oligonucléotide contenant des amines a été dissoute après avoir éliminé les sels, dans les 500 ml de tampon de bicarbonate de sodium 0,25 M (pH 8) et 500 ml d'anhydride succinique 0,1 M dans de l'acétonitrile anhydre ont été ajoutés. Le mélange a été agité pendant 3 h à 37°C. Le mélange réactionnel a été isolé par un procédé de filtration sur gel en utilisant une colonne Sephadex G-25. Le produit final a été analysé en utilisant l'HPLC en phase inverse dans un mode à paires d'ions. Un système de gradient HPLC Waters, une colonne 4 x 250 mm remplie avec Diasorb 130 C16_{T} (7 micron), un éluant : phosphate-K 50 mM (pH 7,0) avec du tétrabutylammonium 2 mM et un gradient d'acétonitrile de 5 à 40 %, un débit de 1 ml/min., une température 45°C ont été utilisés. Il en résultera des oligonucléotides (H') à (O') ayant une fonction carboxyle, localisée sur un insert non-nucléosidique dont l'utilisaltion est possible dans des réactions de liaisons transversales.

### EXEMPLE 5 : Réaction de Couplage d'oligonucléotides ayant une fonction 2'-amine d'un 2'-désoxynucléoside avec des oligonucléotides ayant un groupe carboxyle localisé sur un insert non-nucléosidique dans un duplexe complémentaire.

A. Oligonucléotides formant un duplex à deux brins ayant une liaison covalente entre les deux brins. Les modifications dans les duplex oligonucléotidiques sont opposées l'une à l'autre.
   Le duplexe I à liaison transversale 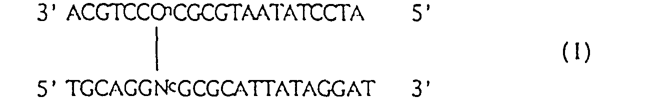
3' ACGTCCOⁿCGCGTAATATCCTA 5'

  (I)

5' TGCAGGNᶜGCGCATTATAGGAT 3'
 dans lequel N^{c} représente un insert non-nucléosidique ayant un groupe carboxyle, "I" représente la liaison transversale entre les fonctions carboxyle et amine des brins oligonucléotidiques, est préparé par une réaction réalisée dans du tampon MES 0,05 M (pH 6,7) avec MgCl₂ 0,02 M. On a dissous 0,1 unité d'A₂₆₀ des deux oligomères (A) et (H') dans 37,5 ml de tampon, le mélange a été chauffé à 95°C, puis refroidi lentement et on a ajouté 37,5 ml de CDI (carbodiimide) 0,4 M dans le même tampon. Après 120 h, le mélange réactionnel, a été analysé et le produit final a été isolé à l'aide d'une HPLC en phase inverse dans un mode à paires d'ions comme décrit ci-dessus.
   La production du duplex à liaison transversale (I) a été obtenue avec un rendement de 26 %.
   Selon une procédure similaire, des duplex à liaison transversale ont été préparés avec des productions s'étendant de 56 % à 82 %. 
5'    CGG TN^cG AGG GGA CTT TCC GAG TGG C    3'
            |
3'    GCC AU^nC TCC CCT GAA AGG CTC ACC G    5'    (11)
 
5'  CGG TAG AGG GGA CTT TCC GANc TGG C  3'
                                                    |
3'  CGG ATC TCC CCT GAA AGG CTUn ACC G  5'  (III)
 
5'   CGG TN:G AGC TCA CTT TCC GAG TGG C _   3'
          |
3'   GCC AUⁿC TCG AGT GAA AGG CTC ACC G   5'   (IV)
 
5' CGG TAG AGC TCA CTT TCC GAN^c TGG C 3'
3' GCC ATC TCG AGT GAA AGG CTU^n ACC G 5' (V)
B. Oligonucléotides formant un duplex à deux brins ayant deux liaisons covalentes entre les chaînes. Les modifications dans les duplex oligonucléotidiques sont opposées l'une à l'autre.
   Les duplexes à deux liaisons transversales 
5'     CGC TNᶜG  AGG  GGA  CTT  TCC  GANᶜ  TGG  C  3'
              |                                  |
3'     GCC AUⁿC  TCC  CCT  GAA AGG  CTUⁿ  ACC  G  5'  (VI)
 
5'    CGG TN^cG  AGC  TCA  CTT  TCC  GAN^c  TGG  C    3'
3'    GCC AU^nC  TCG  AGT  GAA  AGG  CTU^n  ACC  G    5'  (VII)
 dans lesquels N^{c} représente un insert non-nucléosidique ayant un groupe carboxyle, "I" représente la liaison transversale entre les fonctions carboxyle et amine des brins oligonucléotidiques, sont préparés de manière similaire à l'Exemple 5.A. Les productions des duplexes (VI) et (VII) sont de 57 % et 84 % respectivement.
   Il est possible de démontrer la présence d'un duplexe à liaison transversale avec deux liaisons covalentes à l'aide de l'endocucléase de restriction Alu I, qui reconnaît la séquence double brin 5'... AGCT...3'. Comme témoin nous utilisons les duplex à liaison transversale (II) et (III) possédant une liaison transversale.
C. Oligonucléotides formant un duplex à deux brins ayant une liaison covalente entre les chaines. La modification dans l'un des oligonucléotides du duplex est décalée par rapport à l'autre de deux nucléotides.
   duplexe I à liaison transversale décalée 
5' CGG TAG N^cGG GGA CTT TCC GAG TGG C 3'
3' GCC AU^nC TCC CCT GAA AGG CTC ACC G 5' (VIII)
   La production de (VIII) est de 84 %.

### EXEMPLE 6 : Stabilité des duplexes

Les courbes de fusion thermique ont été déterminées en utilisant un spectrophotomètre 150-20 (Hitachi, Japon). Pour déterminer les valeurs Tₘ, la dérivée première a été calculée. Le comportement en fusion des duplex à liaison transversale a été déterminée dans le tampon MES 0,05 M (pH 6,5, MgCl₂ 0,02 M). A des fins de comparaison, la stabilité thermique des duplex naturels de structure analogue et des duplex composés de précurseurs oligonucléotidiques modifiés a été étudié.

| Numéro du duplex | Type de duplex | Tₘ, °C |
|---|---|---|
| | - naturel | 63 |
| | - non-lié | 34 |
| I | - lié de manière | 68 |
| | transversale | |
| | - naturel | 77 |
| | - non-lié | 74 |
| II | - lié de manière | 85 |
| | transversale | |
| | - naturel | 77 |
| | - non-lié | 74 |
| VIII | - lié de manière | 86 |
| | transversale | |

La plus forte Tₘ des duplexes à liaison(s) transversale(s) a confirmé la stabilisation résultant de la présence de la liaison covalente entre les deux brins.

### EXEMPLE 6 : Résistance des duplexes à liaison(s) transversale(s) à des exonucléases spécifiques.

La stabilité à l'hydrolyse du composé résultant de l'action d'un mélange d'une phosphodiestérase de bave d'escargot et d'une phosphatase alcaline a été étudiée dans des conditions strictes d'hydrolyse enzymatique complète (56°C, 180 min.) suivie par une HPLC en phase inverse de l'hydrolysat. Dans ces conditions le duplexe naturel et le mélange de précurseurs oligonucléotidiques modifiés (oligomères (A) et (H)) ont été hydrolysés complètement alors que le duplexe "lié" (I) a été hydrolysé à 30 % seulement. Cette faible hydrolyse est indubitablement un résultat de la liaison covalente présente entre les oligonucléotides composant le duplexe "lié" (I)

## Revendications

1. Oligonucléotide double brin ou simple brin comportant une ou plusieurs liaison(s) covalente(s) transversale(s) respectivement inter ou intra oligonucléotidique(s) caractérisé en ce que la ou chaque liaison covalente est constituée par un lien amide entre un groupe amine primaire d'un brin et un groupe carboxyle de l'autre brin ou du même brin respectivement, ledit groupe amine primaire étant substitué directement en position 2' du cycle osidique d'un nucléotide d'un brin et, ledit groupe carboxyle étant porté par un groupe aliphatique espaceur substitué sur un nucléotide ou un insert non nucléotidique qui comporte un chaînon linéaire de 3 atomes de carbone entre les deux liaisons internucléotidiques (analogue de nucléotide) de l'autre brin ou du même brin respectivement.

2. Oligonucléotide selon la revendication 1, caractérisé en ce que ledit groupe carboxyle est porté par un insert non-nucléotidique du type propane-diol-1,3 substitué en position 2 par un groupe aliphatique portant à son extrêmité ledit groupe carboxyle.

3. Oligonucléotide selon la revendication 1, caractérisé en ce que ledit groupe carboxyle est porté par un groupe aliphatique substitué en position 2' d'un 2' désoxynucléotide.

4. Oligonucléotide selon l'une des revendications 1 à 3 caractérisé en ce que le(s) nucléotide(s) et analogue de nucléotide impliqués dans une liaison covalente inter ou intra moléculaire sont en position d'appariement.

5. Oligonucléotide selon l'une des revendications 1 à 3 caractérisé en ce que le(s) nucléotide(s) et analogue de nucléotide impliqués dans une liaison covalente ne sont pas en position d'appariement.

6. Oligonucléotide selon la revendication 5 caractérisé en ce que le(s) nucléotide(s) et analogue de nucléotide impliqués dans une liaison covalente sont décalés de 1 à 5 nucléotides par rapport à la position d'appariement.

7. Oligonucléotide selon l'une des revendications 1 ou 2, caractérisé en ce que ledit groupe aliphatique comporte une chaine linéaire de 3 à 23 atomes entre ledit lien amide et le site du nucléotide ou analogue de nucléotide sur lequel il est substitué.

8. Oligonucléotide selon la revendication 7 caractérisé en ce que ledit groupe aliphatique comporte de 7 à 16 atomes.

9. Oligonucléotide selon la revendication 8 caractérisé en ce que ledit groupe aliphatique comporte 9 atomes ct le nucléotide et analogue de nucléotide impliqués dans la liaison covalente sont décalés de nucléotides par rapport à la position d'appariement.

10. Oligonucléotide selon l'une des revendications 1 à 9 caractérisé en ce que ledit groupe aliphatique espaceur substitué par un groupe carboxyle, résulte de l'acylation par un anhydride d'acide d'un groupe aminé primaire substitué directement en position 2' d'un nucléotide ou en position 2 d'un analogue de nucléotide.

11. Oligonucléotide selon l'une des revendications 1 à 9 caractérisé en ce que ledit groupe aliphatique espaceur substitué par un groupe carboxyle résulte de l'acylation d'un groupe amine substitué directement en position 2' sur un nucléotide ou sur un analogue de nucléotide en position 2, par un acide aminé dont l'extrémité NH ₂ est elle-même acylée par un anhydride d'acide.

12. Oligonucléotide selon la revendication 11, caractérisé en ce que l'acide aminé est la β-alanine.

13. Oligonucléolide selon l'une des revendications 10 à 12, caractérisé en ce que l'anhydride d'acide est un anhydride cyclique tel que l'anhydride succinimique ou l'anhydride tartarique.

14. Oligonucléotide selon l'une des revendications 1 à 13 caractérisé en ce qu'un reste monovalent en position 2' d'un 2' désoxynucléotide d'un brin est relié au reste monovalent en position 2 d'un analogue du type 1,3 propane diol d'un autre brin ou du même brin par un reste divalent de formule 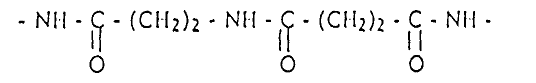 ou

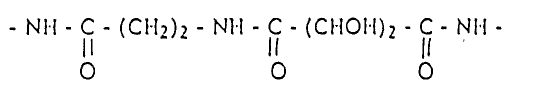

15. Synthon non nucléotidique utile dans la préparation d'un oligonucléotide selon l'une des revendications 1 à 14, caractérisé en ce qu'il est constitué par un composé propane-diol dont les fonctions hydroxyle en positions 1 et 3 sont protégées par les groupes protecteurs conventionnels des fonctions 5' et 3' de nucléotides dans les réactions de synthèse d'oligonucléotide et la position 2 est substituée directement par un groupe NH₂ ou un reste acyl d'acide aminé dont le groupe NH₂ libre est protégé par un groupe trifluoroacétyle.

16. Synthon non nucléotidique selon la revendication 15 caractérisé en ce qu'il répond à la formule 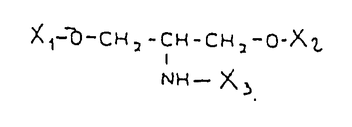 où X₁ et X₂ sont des groupes protecteurs conventionnels des groupes OH 5' et 3' d'un nucléotide, utilisés dans les procédés de synthèse d'oligonucléotide. X₃ est un groupe protecteur de groupe amine ou un reste acyl d'acide aminé dont le groupe NH₂ est protégé par un groupe protecteur de groupe amine.

17. Synthon non nucléotidique selon la revendicaltion 16 caractérisé en ce qu'il répond à la formule

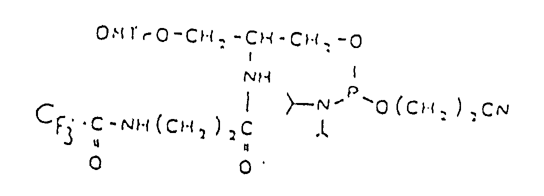

18. Procédé de préparation d'un oligonucléotide selon l'une des revendications 1 à 14, caractérisé en ce qu'on réalise une liaison covalence par couplage d'un groupe amine primaire d'un brin et un groupe carboxyle de l'autre brin ou d'une autre partie du même brin, par réaction de condensation à pH 6,5 en présence d'un agent de condensation.

19. Procédé selon la revendication 18, caractérisé en ce que l'agent de condensation est du carbodiimide soluble dans l'eau.

20. Procédé, de préparation d'un oligonucléotide selon l'une des revendications 18 ou 19, caractérisé en ce qu'on prépare le brin comportant un groupe carboxyle à partir d'un brin comportant un groupe amine obtenu à l'aide d'un synthon nucléotidique comportant les groupes hydroxyle des extrémités 5' et 3' convenablement protégées pour être utilisé dans un procédé de synthèse automatique ou manuelle d'acides nucléiques et un goupe amine substitué en position 2' protégé par un groupe trifluoroacétyle ou d'un synthon non nucléotidique selon l'une des revendications 15 à 17 dont on déprotége ledit groupe amine puis que l'on couple à un anhydride d'acide comportant undit groupe aliphatique.

21. Procédé selon la revendication 20, caractérisé en ce qu'on utilise un synthon nucléotidique dans lequel lesdits groupes hydroxyle sont protégés par des groupes protecteurs utilisés dans les procédés de synthèse d'oligonucléotides au phosphoramidite.

22. Procédé selon la revendication 21, caractérisé en ce qu'on utilise un synthon nucléotidique qui répond à la formule

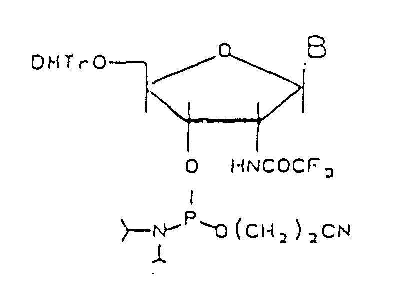

## Claims

1. Double-stranded or single-stranded oligonucleotide containing one or more inter- or intra-oligonucleotide covalent crosslink(s), respectively, characterized in that the or each covalent link consists of an amide linkage between a primary amine group of one strand and a carboxyl group of the other strand or of the same strand, respectively, said primary amine group being substituted directly at the 2' position of the saccharide ring of a nucleotide of one strand, and said carboxyl group being carried by an aliphatic spacer group substituted on a nucleotide or a non-nucleotide insert which contains a linear motif of 3 carbon atoms between the two internucleotide links (nucleotide analogue) of the other strand or of the same strand, respectively.

2. Oligonucleotide according to claim 1, characterized in that said carboxyl group is carried by a non-nucleotide insert of the 1,3-propanediol type substituted at the 2 position with an aliphatic group carrying said carboxyl group at its end.

3. Oligonucleotide according to claim 1, characterized in that said carboxyl group is carried by an aliphatic group substituted at the 2' position of a 2'-deoxynucleotide.

4. Oligonucleotide according to one of claims 1 to 3, characterized in that the nucleotide (s) and nucleotide analogue involved in an inter- or intramolecular covalent link are in a pairing position.

5. oligonucleotide according to one of claims 1 to 3, characterized in that the nucleotide(s) and nucleotide analogue involved in a covalent link are not in a pairing position.

6. Oligonucleotide according to claim 5, characterized in that the nucleotide(s) and nucleotide analogue involved in a covalent link are offset by 1 to 5 nucleotides relative to the pairing position.

7. Oligonucleotide according to either of claims 1 and 2, characterized in that said aliphatic group contains a linear chain of 3 to 23 atoms between said amide linkage and the site of the nucleotide or nucleotide analogue on which it is substituted.

8. Oligonucleotide according to claim 7, characterized in that said aliphatic group contains from 7 to 16 atoms.

9. Oligonucleotide according to claim 8, characterized in that said aliphatic group contains 9 atoms, and the nucleotide and nucleotide analogue involved in the covalent link are offset by 2 nucleotides relative to the pairing position.

10. Oligonucleotide according to one of claims 1 to 9, characterized in that said aliphatic spacer group substituted with a carboxyl group results from the acylation with an acid anhydride of a primary amine group substituted directly at the 2' position of a nucleotide or at the 2 position of a nucleotide analogue.

11. Oligonucleotide according to one of claims 1 to 9, characterized in that said aliphatic spacer group substituted with a carboxyl group results from the acylation of an amine group substituted directly at the 2' position on a nucleotide or on a nucleotide analogue at the 2 position with an amino acid whose NH₂ end is itself acylated with an acid anhydride.

12. Oligonucleotide according to claim 11, characterized in that the amino acid is β-alanine.

13. Oligonucleotide according to one of claims 10 to 12, characterized in that the acid anhydride is a cyclic anhydride such as succinimic anhydride or tartaric anhydride.

14. Oligonucleotide according to one of claims 1 to 13, characterized in that a monovalent residue at the 2' position of a 2'-deoxynucleotide of one strand is linked to the monovalent residue at the 2 position of an analogue of the 1,3-propanediol type of another strand or of the same strand via a divalent residue of formula 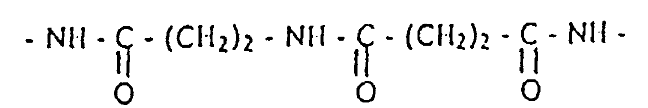 or

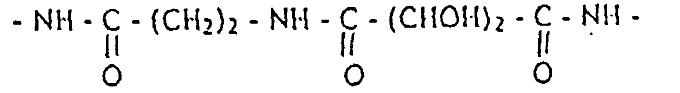

15. Non-nucleotide synthon which is of use in the preparation of an oligonucleotide according to one of claims 1 to 14, characterized in that it consists of a propanediol compound the hydroxyl functions of which at the 1 and 3 positions are protected by the conventional protective groups for the 5' and 3' functions of nucleotides in oligonucleotide synthesis reactions, and the 2 position of which is substituted directly with an NH₂ group or an amino acid acyl residue whose free NH₂ group is protected by a trifluoroacetyl group.

16. Non-nucleotide synthon according to claim 15, characterized in that it corresponds to the formula 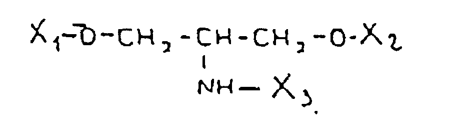 where X₁ and X₂ are conventional protective groups for the 5'- and 3'-OH groups of a nucleotide, used in the methods of oligonucleotide synthesis. X₃ is an amine group-protecting group or an amino acid acyl residue whose NH₂ group is protected by an amine group-protecting group.

17. Non-nucleotide synthon according to claim 16, characterized in that it corresponds to the formula

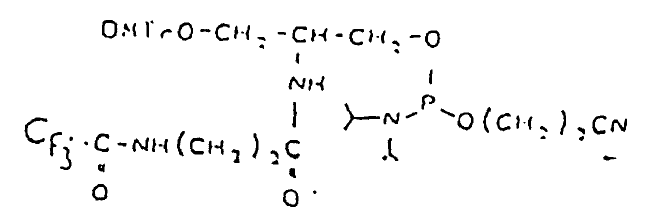

18. Method for preparing an oligonucleotide according to one of claims 1 to 14, characterized in that a covalent link is produced by coupling a primary amine group of one strand and a carboxyl group of the other strand or of another portion of the same strand, by a condensation reaction at pH 6.5 in the presence of a condensing agent.

19. Method according to claim 18, characterized in that the condensing agent is water-soluble carbodiimide.

20. Method for preparing an oligonucleotide according to either of claims 18 and 19, characterized in that the strand containing a carboxyl group is prepared from a strand containing an amine group, obtained using a nucleotide synthon containing the hydroxyl groups of the 5' and 3' ends suitably protected in order for it to be used in a method of automatic or manual synthesis of nucleic acids and an amine group substituted at the 2' position protected by a trifluoroacetyl group, or using a non-nucleotide synthon according to one of claims 15 to 17, said amine group of which is deprotected and which is then coupled to an acid anhydride containing a said aliphatic group.

21. Method according to claim 20, characterized in that a nucleotide synthon is used in which said hydroxyl groups are protected by protective groups used in the phosphoramidite methods of oligonucleotide synthesis.

22. Method according to claim 21, characterized in that a nucleotide synthon which corresponds to the following formula is used

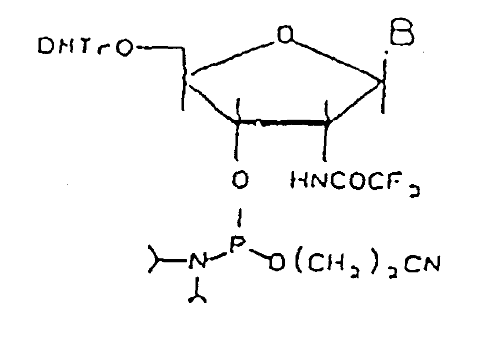

## Patentansprüche

1. Duplex- oder Simplexoligonukleotid mit einer querverlaufenden kovalenten Bindung bzw. mehreren querverlaufenden kovalenten Bindungen zwischen den Oligonukleotiden bzw. innerhalb der Oligonukleotide, dadurch gekennzeichnet, daß jede kovalente Bindung aus einer Amidbindung zwischen einer primären Aminogruppe eines Strangs und einer Carboxylgruppe des anderen Strangs bzw. des gleichen Strangs besteht, wobei diese primäre Aminogruppe direkt in 2'-Stellung des Ose-Rings eines Nukleotids eines Strangs substituiert ist, wobei die Carboxylgruppe von einer aliphatischen Spacer-Gruppe getragen wird, die an einem Nukleotid oder einer nichtnukleotidischen Insertion mit einer linearen Kette mit drei Kohlenstoffatomen zwischen den beiden Bindungen zwischen den Nukleotiden (Nukleotidanalog) des anderen Strangs bzw. des gleichen Strangs substituiert ist.

2. Oligonukleotid nach Anspruch 1, dadurch gekennzeichnet, daß die Carboxylgruppe von einer nichtnukleotidischen Insertion getragen wird, wobei die nichtnukleotidische Insertion dem Typ Propan-1,3-diol, das in 2-Stellung mit einer aliphatischen Gruppe, die an ihrem Terminus diese Carboxylgruppe trägt, substituiert ist, entspricht.

3. Oligonukleotid nach Anspruch 1, dadurch gekennzeichnet, daß die Carboxylgruppe von einer aliphatischen Gruppe, die in 2'-Stellung mit einem 2'-Desoxynukleotid substituiert ist, getragen wird.

4. Oligonukleotid nach einem der Ansprüche 1 bis 3, dadurch gekennzeichnet, daß das Nukleotid bzw. die Nukleotide und das Nukleotidanalog, die an einer inter-oder intramolekularen kovalenten Bindung beteiligt sind, gepaart vorliegen.

5. Oligonukleotid nach einem der Ansprüche 1 bis 4, dadurch gekennzeichnet, daß das Nukleotid bzw. die Nukleotide und das Nukleotidanalog, die an einer kovalenten Bindung beteiligt sind, nicht gepaart vorliegen.

6. Oligonukleotid nach Anspruch 5, dadurch gekennzeichnet, daß das Nukleotid bzw. die Nukleotide und das Nukleotidanalog, die an einer kovalenten Bindung beteiligt sind, in bezug auf die Paarung um 1 bis 5 Nukleotide versetzt sind.

7. Oligonukleotid nach Anspruch 1 oder 2, dadurch gekennzeichnet, daß die aliphatische Gruppe zwischen der Amidbindung und der Lage des Nukleotids oder Nukleotidanalogs, auf dem sie substituiert ist, eine gerade Kette mit 3 bis 23 Atomen aufweist.

8. Oligonukleotid nach Anspruch 7, dadurch gekennzeichnet, daß die aliphatische Gruppe 7 bis 16 Atome aufweist.

9. Oligonukleotid nach Anspruch 8, dadurch gekennzeichnet, daß die aliphatische Gruppe 9 Atome aufweist und das Nukleotid und Nukleotidanalog, die an der kovalenten Bindung beteiligt sind, in bezug auf die Paarung um 2 Nukleotide versetzt sind.

10. Oligonukleotid nach einem der Ansprüche 1 bis 9, dadurch gekennzeichnet, daß die durch eine Carboxylgruppe substituierte aliphatische Spacer-Gruppe durch Acylierung einer direkt in 2'-Stellung eines Nukleotids oder in 2-Stellung eines Nukleotidanalogs substituierten primären Aminogruppe mit einem Säureanhydrid entsteht.

11. Oligonukleotid nach einem der Ansprüche 1 bis 9, dadurch gekennzeichnet, daß die durch eine Carboxylgruppe substituierte aliphatische Spacer-Gruppe durch Acylierung einer direkt in 2'-Stellung eines Nukleotids oder in 2-Stellung eines Nukleotidanalogs substituierte Aminogruppe mit einer Aminosäure, deren NH₂-Terminus wiederum selbst mit einem Säureanhydrid acyliert ist, entsteht.

12. Oligonukleotid nach Anspruch 11, dadurch gekennzeichnet, daß es sich bei der Aminosäure um β-Alanin handelt.

13. Oligonukleotid nach einem der Ansprüche 10 bis 12, dadurch gekennzeichnet, daß es sich bei dem Säureanhydrid um ein cyclisches Anhydrid wie Bernsteinsäureanhydrid oder Weinsäureanhydrid handelt.

14. Oligonukleotid nach einem der Ansprüche 1 bis 13, dadurch gekennzeichnet, daß ein einwertiger Rest in 2'-Stellung eines 2'-Desoxynukleotids durch einen zweiwertigen Rest der Formel 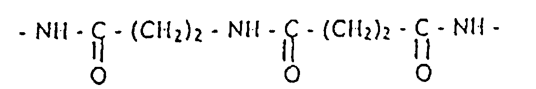 oder 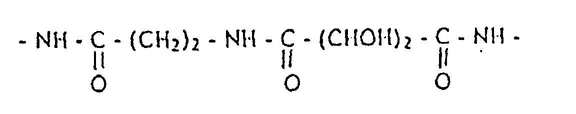 an den einwertigen Rest in 2-Stellung eines Analogs der Art Propan-1,3-diol gebunden ist.

15. Nichtnukleotid-Synthon, das sich zur Herstellung eines Oligonukleotids nach einem der Ansprüche 1 bis 14 eignet, dadurch gekennzeichnet, daß es aus einer Propandiolverbindung besteht, in welcher die Hydroxylfunktionen in 1- und 3-Stellung durch übliche Schutzgruppen der 5'- und 3'-Funktionen von Nukleotiden in Oligonukleotidsynthesereaktionen geschützt sind und die 2-Stellung direkt durch eine NH₂₋Gruppe oder einen Aminosäurerest, deren freie NH₂₋Gruppe durch eine Trifluoracetylgruppe geschützt ist, substituiert ist.

16. Nichtnukleotid-Synthon nach Anspruch 15, dadurch gekennzeichnet, daß es der Formel 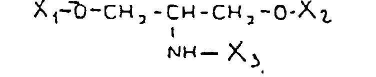 entspricht, in der X₁ und X₂ übliche Schutzgruppen für die 5'- und 3'-OH-Gruppen eines Nukleotids, die bei Oligonukleotidsyntheseverfahren verwendet werden, darstellen und X₃ eine Aminogruppenschutzgruppe oder einen Aminosäureacylrest, dessen NH₂-Gruppe durch eine Aminogruppenschutzgruppe geschützt ist, darstellt.

17. Nichtnukleotid-Synthon nach Anspruch 16, dadurch gekennzeichnet, daß es der Formel 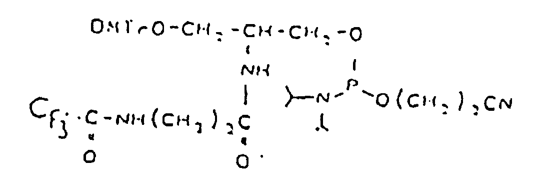 entspricht.

18. Verfahren zur Herstellung eines Oligonukleotids nach einem der Ansprüche 1 bis 14, dadurch gekennzeichnet, daß man durch Kupplung einer primären Aminogruppe eines Strangs und einer Carboxylgruppe des anderen Strangs oder eines anderen Teils des gleichen Strangs durch Kondensation bei pH 6,5 in Gegenwart eines Kondensationsmittels eine kovalente Bindung herstellt.

19. Verfahren nach Anspruch 18, dadurch gekennzeichnet, daß es sich bei dem Kondensationsmittel um wasserlösliches Carbodiimid handelt.

20. Verfahren zur Herstellung eines Oligonukleotids nach Anspruch 18 oder 19, dadurch gekennzeichnet, daß man den Strang, der eine Carboxylgruppe aufweist, aus einem Strang, der eine Aminogruppe aufweist, wobei letztere mit Hilfe eines Nukeotid-Synthons erhalten wurde, das die OH-Gruppen der 5'- und 3'-Termini zweckmäßigerweise geschützt aufweist, um in einem automatischen oder manuellen Verfahren zur Herstellung von Nukleinsäuren verwendet werden zu können, und das eine Aminogruppe aufweist, die in 2'-Stellung durch eine Trifluoracetylgruppe substituiert ist, oder aus einem Nukleotid-Synthon nach einem der Ansprüche 15 bis 17, dessen Aminogruppe entschützt wird und dann mit einem Säureanhydrid, das eine besagte aliphatische Gruppe aufweist, gekoppelt wird, herstellt.

21. Verfahren nach Anspruch 20, dadurch gekennzeichnet, daß man ein Nukleotid-Synthon verwendet, in dem die besagten Hydroxylgruppen durch Schutzgruppen geschützt sind, die bei den Phosphoramidit-Verfahren der Oligonukleotidsynthese verwendet werden.

22. Verfahren nach Anspruch 21, dadurch gekennzeichnet, daß man ein Nukleotid-Synthon verwendet, das der Formel 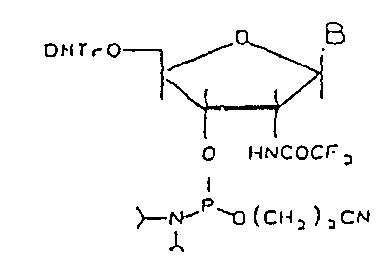 entspricht.
